# EUROPEAN PATENT SPECIFICATION

(11) **EP 1 338 654 B1**
(45) Date of publication and mention of the grant of the patent: **15.10.2008**
(21) Application number: 01980986.2
(22) Date of filing: 06.11.2001
(51) Int. Cl.: C12P 19/38, C12N 15/09

(54) **PROCESS FOR PRODUCING NUCLEOSIDE COMPOUND**
VERFAHREN ZUR HERSTELLUNG EINER NUKLEOSIDVERBINDUNG
PROCEDE DE PRODUCTION DE COMPOSE NUCLEOSIDE

(30) Priority: 06.11.2000 JP 2000337715; 14.12.2000 JP 2000380576; 22.03.2001 JP 2001082857
(43) Date of publication of application: 27.08.2003
(60) Divisional application: 07022944.8
(73) Proprietor: Mitsui Chemicals, Inc., Tokyo (JP)
(72) Inventor: ARAKI, Tadashi, Mobara-shi, Chiba 297-0117 (JP); IKEDA, Ichiro, Mobara-shi, Chiba 297-0117 (JP); TAKAHASHI, Katsuyuki, Mobara-shi, Chiba 297-0117 (JP); ITO, Kiyoshi, Mobara-shi, Chiba 297-0117 (JP); ASANO, Tamotsu, Mobara-shi, Chiba 297-0117 (JP); NIKUMARU, Seiya, Mobara-shi, Chiba 297-0117 (JP); NAKAMURA, Takeshi, Mobara-shi, Chiba 297-0117 (JP); ISHIBASHI, Hiroki, Omuta-shi, Fukuoka 836-8610 (JP); NAGAHARA, Kiyoteru, Omuta-shi, Fukuoka 836-8610 (JP); FUKUIRI, Yasushi, Omuta-shi, Fukuoka 836-8610 (JP)
(74) Representative: Stuart, Ian Alexander
(86) International application number: PCT/JP2001/009701
(87) International publication number: WO 2002/036800

(56) References cited:
- EP-A1- 0 896 065
- EP-A2- 0 198 387
- WO-A1-00/70074
- JP-A- 1 320 995
- JP-A- 9 215 498
- JP-A- 11 137 290
- JP-A- 59 213 397
- PATENT ABSTRACTS OF JAPAN vol. 013, no. 326 (C-620), 24 July 1989 (1989-07-24) & JP 01 104190 A (AJINOMOTO CO INC), 21 April 1989 (1989-04-21)

### SEQUENCE LISTING

<110> Mitsui Chemicals, Inc.
<120> Process for Producing Nucleoside Compound
<130> IS/FP6144679
<140> EP 01980986.2
   <141> 2001-11-06
<150> PCT/JP01/09701
   <151> 2001-11-06
<150> JP20000337715
   <151> 2000-11-06
<150> JP20010082857
   <151> 2001-03-22
<150> JP20000380576
   <151> 2000-12-14
<160> 2
<170> PatentIn version 3.1
<210> 1
   <211> 30
   <212> DNA
   <213> Artificial sequence
<220>
   <221> source
   <223> /note = "Description of artificial sequence: Oligonucleotide to act as a PCR primer for cloning of purine nucleoside phosphorylase of E. coli K12"
<400> 1
   gtgaattcac aaaaaggata aaacaatggc 30
<210> 2
   <211> 29
   <212> DNA
   <213> Artificial sequence
<220>
   <221> source
   <223> /note = "Description of artificial sequence: Oligonucleotide to act as a PCR primer for cloning of purine nucleoside phosphorylase of E. coli K12"
<400> 2
   tcgaagcttg cgaaacacaa ttactcttt 29

### SEQUENCE LISTING

<110> Mitsui Chemicals, Inc.
<120> Process for Producing Nucleoside Compound
<130> IS/FP6144679
<140> EP 01980986.2
   <141> 2001-11-06
<150> PCT/JP01/09701
   <151> 2001-11-06
<150> JP20000337715
   <151> 2000-11-06
<150> JP20010082857
   <151> 2001-03-22
<150> JP20000380576
   <151> 2000-12-14
<160> 2
<170> PatentIn version 3.1
<210> 1
   <211> 30
   <212> DNA
   <213> Artificial sequence
<220>
   <221> source
   <223> /note = "Description of artificial sequence: Oligonucleotide to act as a PCR primer for cloning of purine nucleoside phosphorylase of E. coli K12"
<400> 1
   gtgaattcac aaaaaggata aaacaatggc 30
<210> 2
   <211> 29
   <212> DNA
   <213> Artificial sequence
<220>
   <221> source
   <223> /note = "Description of artificial sequence: Oligonucleotide to act as a PCR primer for cloning of purine nucleoside phosphorylase of E. coli K12"
<400> 2
   tcgaagcttg cgaaacacaa ttactcttt 29

### SEQUENCE LISTING

<110>MITSUI CHEMICALS, INC.
<120>Method of producing a nucleoside compound
<130>MTC01P254
<160>2
<210>1
   <211>30
   <212>DNA
   <213>Artificial Sequence
<220>
   <223>Oligonucleotide to act as a PCR primer for cloning of purine nucleoside phosphorylase of E.coli K-12
<400>1
   gtgaattcac aaaaaggata aaacaatggc 30
<210>2
   <211>29
   <212>DNA
   <213>Artificial Sequence
<220>
   <223>Oligonucleotide to act as a PCR primer for cloning of purine nucleoside phosphorylase of E.coli K-12
<400>2
   tcgaagcttg cgaaacacaa ttactcttt 29

## Description

### Technical Field

The present invention relates to a process for producing a nucleoside compound using a nucleoside phosphorylase. Various nucleosides and analogue compounds thereof are used as raw materials for medicines such as antiviral medicine, anticancer medicine or antisense medicine, or as a component used in such medicines.

### Background Art

Nucleoside phosphorylase is a generic term for enzymes which give rise to phosphorolysis of the N-glycoside bond of a nucleoside in the presence of phosphoric acid, and, in the case of ribonucleosides, catalyze a reaction represented by the following formula:

ribonucleoside + phosphoric acid (salt) → nucleic acid base + ribose 1-phosphoric acid

The above enzymes, which are mainly divisible into purine nucleoside phosphorylases and pyrimidine nucleoside phosphorylases, occur widely in the biological world, and are present in the tissues of mammals, birds and fishes, as well as in yeasts and bacteria. The above enzyme reaction is reversible and synthesis of various nucleoside compounds utilizing the reverse reaction has been known heretofore.

There are known, for example, processes for producing thymidine (JP-A-01-104190), 2'-deoxyadenosine (JP-A-11-137290) or 2'-deoxyguanosine (JP-A-11-137290) from 2'-deoxyribose 1-phosphoric acid and a nucleic acid base (thymine, adenine or guanine). Further, a technique was reported in Agric., Biol., Chem., Vol. 50 (1), pp. 121-126, (1986), including the steps of decomposing inosine into ribose 1-phosphoric acid and hypoxanthine through a reaction in the presence of phosphoric acid using a purine nucleoside phosphorylase derived from Enterobacter aerogenes, and producing ribavirin, as an anti-viral agent, from the ribose 1-phosphoric acid, which is isolated by an ion exchange resin and 1,2,4-triazole-3-carboxamide in the presence of the same purine nucleoside phosphorylase derived from Enterobacter aerogenes.

However, the reaction for producing a nucleoside compound from either pentose-1-phosphoric acid or a salt thereof and a nucleoside acid base by using the above reverse reaction in the presence of the enzyme leads to an equilibrium state, so that the reaction has had a technical drawback of low conversion.

### Disclosure of the Invention

The object to be achieved by the present invention is to provide, a simple method for allowing a reaction to proceed at a high conversion and a means contributing to a reduction in raw material cost and an improvement of product purity, in producing a nucleoside compound industrially.

Therefore, the object of the present invention is to provide a simple and widely-applicabled process for producing a nucleoside compound, which comprises a step of reacting pentose-1-phosphoric acid with a nucleic acid base or a nucleic acid base analogue in the presence of nucleoside phosphorylase activity, wherein the nucleoside compound is produced at a high conversion rate.

The present inventors made an intensive study in order to achieve the above object. As a result, the present inventors found that, when a metal cation capable of forming a sparingly water-soluble salt with phosphoric acid ion is allowed to be present in the reaction mixture, the phosphoric acid ion formed as a by-product of reaction is precipitated as a sparingly water-soluble salt and removed out of the reaction system and, as a result, the equilibrium of reaction moves to a direction of nucleoside compound synthesis and a conversion rate of reaction increased.

The present inventors further found that, when the metal cation capable of forming a sparingly water-soluble salt with phosphoric acid is allowed to be present in the reaction mixture in the form of a salt of pentose-1-phosphoric acid, there can be obtained the same effect as when the metal cation capable of forming a sparingly water-soluble salt with phosphoric acid ion, or a salt thereof is added.

The present inventors also found that in a process for producing a nucleoside compound by subjecting pentose-1-phosphoric acid to an exchange reaction of phosphoric acid group and either nucleic acid base or nucleic acid base analogue in the presence of nucleoside phosphorylase, when a nucleic acid base analogue, which has generally a low solubility and a low reactivity, is introduced into the reaction system in the form of an aqueous alkali metal solution, the substrate concentration during reaction can be made high and, therefore, a nucleoside compound can be obtained at a high conversion rate.

It was also found that, regarding the production of a nucleoside compound, when there is used, as the aqueous alkali metal solution, an aqueous solution of a salt of a metal cation containing a metal cation containing lithium ion or magnesium ion, the phosphoric acid formed by the reaction is insolubilized in the form of lithium phosphate or magnesium phosphate; thereby, the equilibrium of enzyme reaction is directed to a side of nucleoside compound synthesis; as a result, a nucleoside compound is obtained at a high selectivity and high yield which have been unattainable with conventional art.

It was also found that, when a sparingly water-soluble compound of a metal cation, such as magnesium hydroxide, is particularly used as the source of the metal cation capable of forming a sparingly water-soluble salt with phosphoric acid, various advantages are obtained. For example, since there is no increase in the salt concentration of the reaction mixture, a reduction of the reaction rate is prevented, which is caused by increase of the salt concentration in the reaction mixture. Since such a base of a metal cation can be added in one portion (total amount) before the reaction, the operation of reaction is simpler than when an aqueous solution of magnesium is added. The filtration efficiency for the reaction mixture obtained is improved strikingly.

The present inventors also found that, when magnesium hydroxide is used as the source of a metal cation, a crystal of sparingly water-soluble phosphate (magnesium phosphate) formed as a by-product of reaction has a size and shape relatively suited for filtration of the reaction mixture, which enables quick filtration of the reaction mixture conducted after the completion of reaction.

In order to produce a nucleoside compound industrially, it is necessary to increase the volumetric efficiency and the recovery during purification and, therefore, it is desired to accumulate the produced nucleoside in the reaction mixture at a high concentration. When, as in the above, a metal cation is allowed to be present in the reaction mixture to react it with phosphoric acid ion (which is generated with proceeding of reaction) to precipitate the resulting substance as a sparingly water-soluble salt and when the amounts of individual components are increased in order to obtain a high concentration of a nucleoside compound, the pH of reaction mixture decreases as the reaction of phosphoric acid and metal cation proceeds and the resulting sparingly water-soluble salt precipitates, and too low a pH results in a low conversion of reaction. This low conversion of reaction caused by reduction in pH of the reaction mixture is considered to be due to, for example, the decomposition of formed nucleoside compound and the partial loss of enzymatic activity.

In such a case, the pH of reaction mixture is preferred to be controlled in order to maintain a desired conversion of reaction. However, when the initial (at the start of reaction) feed amounts are increased in order to obtain a nucleoside compound at a high concentration, there were cases that the viscosity of reaction mixture increases with proceeding of reaction, even leading to solidification. Such viscosity increase and solidification of reaction mixture make difficult the above-mentioned control of desired pH. Further, the viscosity increase of reaction mixture requires an excessive power for mixing and kneading of reaction mixture and invites trouble of equipment.

The present inventors found that, when there are used feed amounts necessary for obtaining a nucleoside compound in a reaction mixture at a high concentration, the high viscosity or solidification of reaction mixture can be prevented by adding all or part of the feed amount of at least one component selected from pentose-1-phosphoric acid, either a nucleic acid base or a nucleic acid base analogue and a metal cation, at a particular timing(s) after the start of reaction.

The present invention completed based on the above findings includes the following embodiments:
[1] A process for producing a nucleoside compound, which comprises reacting pentose-1-phosphoric acid with a nucleic acid base or a nucleic acid base analogue in an aqueous reaction medium in the presence of nucleoside phosphorylase activity in the presence of a metal cation capable of forming a sparingly water-soluble salt with phosphoric acid ion. The cation is selected from sodium, potassium, calcium, magnesium, aluminum, barum, iron, cobalt, nickel, copper, silver, molybdenum, lead, zinc and lithium cations. The process is characterized in that, after the start of the reaction (namely, when at least some of the pentose-1-phosphoric acid and at least some of the nucleic acid base or analogue have been added to the reaction medium, and nucleoside phosphorylase activity is present), at least one of component selected from the pentose-1-phosphoric acid, the nucleic acid base or the nucleic acid base analogue, and the metal cation is added to the aqueous reaction medium which is in the midst of the reaction.
[2] A process for producing a nucleoside compound according to the above [1], wherein the pentose-1-phosphoric acid is ribose-1-phosphoric acid or 2-deoxyribose-1-phosphoric acid.
[3] A process for producing a nucleoside compound according to the above [1] or [2], wherein the metal cation capable of forming a sparingly water-soluble salt with phosphoric acid is at least one selected from calcium ion, barium ion, aluminum ion, lithium ion and magnesium ion.
[4] A process for producing a nucleoside compound according to the above [3], wherein the metal cation capable of forming a sparingly water-soluble salt with phosphoric acid is added to the aqueous reaction medium in the form of a metal compound with at least one anion selected from hydroxy ion, chloride ion, and anions of nitric acid, carbonic acid, sulfuric acid, and acetic acid.
[5] A process for producing a nucleoside compound according to the above [3], wherein the metal cation capable of forming a sparingly water-soluble salt with phosphoric acid is added to the aqueous reaction medium in the form of a salt of pentose-1-phosphoric acid.
[6] A process for producing a nucleoside compound according to the above [1], wherein the addition of the at least one component to the aqueous reaction medium from the start of the reaction to the completion of the reaction is made in two or more portions or continuously and gradually.
[7] A process for producing a nucleoside compound according to the above [1] or [6], characterized in that, the amounts of the three components, (i) the pentose-1-phosphoric acid, (ii) the nucleic acid base or the nucleic acid base analogue, and (iii) the metal cation, result in increase of viscosity or solidification of the reaction mixture when the three components are added in one portion, but the increase of viscosity or the solidification can be prevented by adding a part or the whole of the amount of at least one component in a portion(s) from the start of the reaction to completion of the reaction.
[8] A process for producing a nucleoside compound according to any of the above [1] to [7], **characterized in that** the nucleic acid base or the nucleic acid base analogue is added in the form of an aqueous alkali solution.
[9] A process for producing a nucleoside compound according to the above [8], characterized in that the aqueous alkali solution in which the nucleic acid base or the nucleic acid base analogue is dissolved, is a solution containing at least one of lithium hydroxide, lithium carbonate and lithium hydrogencarbonate.

### Best Mode for Carrying Out the Invention

In the present invention, the pentose-1-phosphoric acid is a compound in which phosphoric acid is bonded to the 1-position of a polyhydroxyaldehyde or polyhydroxyketone or a derivative thereof to form an ester bond. As representative examples thereof, there can be mentioned ribose 1-phosphoric acid, 2'-deoxyribose 1-phosphoric acid, 2',3'-dideoxyribose 1-phosphoric acid and arabinose 1-phosphoric acid. However, the pentose-1-phosphoric acid is not restricted thereto.

As the polyhydroxyaldehyde or polyhydroxyketone, there can be mentioned, as natural products, aldopentoses such as D-arabinose, L-arabinose, D-xylose, L-lyxose and D-ribose; ketopentoses such as D-xylulose, L-xylulosel and D-ribulose; and deoxysugars such as D-2-deoxyribose and D-2,3-dideoxyribose. However, the polyhydroxyaldehyde or polyhydroxyketone is not restricted thereto.

The pentose-1-phosphoric acid can be produced by, for example, phosphorolysis of nucleoside compound in the presence of nucleoside phosphorylase (J. Biol. Chem. Vol. 184, 437, 1950) or anomer-selective chemical synthesis.

The nucleic acid base used in the present invention is a nitrogen atom-containing heterocyclic compound which is a structural element of a compound such as DNA or DNA, and is a natural or unnatural nucleic acid base selected from the group consisting of pyrimidines, purines, and base analogues. The base may be substituted with halogen atom, alkyl group, haloalkyl group, alkenyl group, haloalkenyl group, alkynyl group, amino group, alkylamino group, hydroxyl group, hydroxyamino group, aminooxy group, alkoxy group, mercapto group, alkylmercapto group, aryl group, aryloxy group or cyano group.

The halogen atom as a substituent is exemplified by chlorine, fluorine, iodine and bromine. The alkyl group is exemplified by alkyl groups of 1 to 7 carbon atoms such as methyl group, ethyl group or propyl group.

The haloalkyl group is exemplified by haloalkyl groups of 1 to 7 carbon atoms such as fluoromethyl, difluoromethyl, trifluoromethyl, bromomethyl or bromoethyl.

The alkenyl group is exemplified by alkenyl groups of 2 to 7 carbon atoms such as vinyl or allyl. The haloalkenyl group is exemplified by haloalkenyl groups having an alkenyl of 2 to 7 carbon atoms, such as bromovinyl or chlorovinyl. The alkynyl group is exemplified by alkynyl groups of 2 to 7 carbon atoms such as ethynyl or propynyl. The alkylamino group is exemplified by alkylamino groups having an alkyl group of 1 to 7 carbon atoms, such as methylamino or ethylamino. The alkoxy group is exemplified by alkoxy groups of 1 to 7 carbon atoms, such as methoxy or ethoxy. The alkylmercapto group is exemplified by alkylmercapto groups having an alkyl group of 1 to 7 carbon atoms, such as methylmercapto or ethylmercapto. The aryl group is exemplified by phenyl group; alkylphenyl groups having an alkyl group of 1 to 5 carbon atoms, such as methylphenyl or ethylphenyl; alkoxyphenyl groups having an alkoxy group of 1 to 5 carbon atoms, such as methoxyphenyl or ethoxyphenyl; alkylaminophenyl groups having an alkylamino group of 1 to 5 carbon atoms, such as dimethylaminophenyl or diethylaminophenyl; halogenophenyl groups such as chlorophenyl or bromophenyl; and so forth.

As specific examples of the nucleic acid base having a pyrimidine skeleton, there can be mentioned cytosine, uracil, 5-fluorocytosine, 5-fluorouracil, 5-chlorocytosine, 5-chlorouracil, 5-bromocytosine, 5-bromouracil, 5-iodocytosine, 5-iodouracil, 5-methylcytosine, 5-methyluracil (thymine), 5-ethylcytosine, 5-ethyluracil, 5-fluoromethylcytosine, 5-fluorouracil, 5-trifluorocytosine, 5-trifluorouracil, 5-vinyluracil, 5-bromovinyluracil, 5-chlorovinyluracil, 5-ethynylcytosine, 5-ethynyluracil, 5-propynyluracil, pyrimidin-2-one, 4-hydroxyaminopyrimidin-2-one, 4-aminooxypyrimidin-2-one, 4-methoxypyrimidin-2-one, 4-acetoxypyrimidin-2-one, 4-fluoropyrimidin-2-one and 5-fluoropyrimidin-2-one.

As specific examples of the nucleic acid base having a purine skeleton, there can be mentioned purine, 6-aminopurine (adenine), 6-hydroxypurine, 6-fluoropurine, 6-chloropurine, 6-methylaminopurine, 6-dimethylaminopurine, 6-trifluoromethylaminopurine, 6-benzoylaminopurine, 6-acetylaminopurine, 6-hydroxyaminopurine, 6-aminooxypurine, 6-methoxypurine, 6-acetoxypurine, 6-benzoyloxypurine, 6-methylpurine, 6-ethylpurine, 6-trifluoromethylpurine, 6-phenylpurine, 6-mercaptopurine, 6-methylmercaptopurine, 6-aminopurine-1-oxide, 6-hydroxypurine-1-oxide, 2-amino-6-hydroxypyrine (guanine), 2,6-diaminopurine, 2-amino-6-chloropurine, 2-amino-6-iodopurine, 2-aminopurine, 2-amino-6-mercaptopurine, 2-amino-6-methylmercaptopurine, 2-amino-6-hydroxyaminopurine, 2-amino-6-mehtoxypurine, 2-amino-6-benzoyloxypurine, 2-amino-6-acetoxypurine, 2-amino-6-methylpurine, 2-amino-6-cyclopropylaminomethylpurine, 2-amino-6-phenylpurine, 2-amino-8-bromopurine, 6-cyanopurine, 6-amino-2-chloropurine (2-chloroadenine) and 6-amino-2-fluoropurine (2-fluoroadenine).

As specific examples of the base analogue, there can be mentioned 6-amino-3-deazapurine, 6-amino-8-azapurine, 2-amino-6-hydroxy-8-azapurine, 6-amino-7-deazapurine, 6-amino-1-deazapurine and 6-amino-2-azapurine.

In the present invention, the nucleoside compound is a compound in which a nucleic acid base having a pyrimidine skeleton, a nucleic acid base having a purine skeleton, or a base analogue is bonded to the 1-position of the above-mentioned polyhydroxyaldehyde or polyhydroxyketone or its derivative to form an N-glycoside bond. As specific examples, there can be mentioned thymidine, deoxythymidine, deoxyuridine, deoxyguanosine, deoxyadenosine, dideoxyadenosine, trifluorothymidine, ribavirin, orotidine, uracil arabinoside, adenine arabinoside, 2-methyl-adenine arabinoside, 2-chloro-hypoxanthine arabinoside, thioguanine arabinoside, 2,6-diaminopurine arabinoside, cytosine arabinoside, guanine arabinoside, thymine arabinoside, enocitabine, gemcitabine, azidothymidine, idoxuridine, dideoxyadenosine, dideoxyinosine, dideoxycytidine, didehydrodeoxythymidine, thiadideoxycytidine, sorbidine, 5-methyl-uridine, pyrazole, thioinosine, tegafur, doxyfluridine, bredinin, nebularin, allopurinol uracil, 5-fluorouracil, 2'-aminouridine, 2'-aminoadenosine, 2'-aminoguanosine and 2-chloro-2'-aminoinosine.

In the present invention, the nucleoside phosphorylase is a generic term for enzymes which decompose the N-glycoside bond of a nucleoside compound in the presence of phosphoric acid. In the present invention, the reverse reaction can be utilized. The enzyme used in the reaction may be an enzyme of any kind and any origin as long as it has an activity capable of forming an intended nucleoside compound from a corresponding pentose-1-phosphoric acid and a nucleic acid base or a nucleic acid base analogue. The enzyme is largely divided into a purine type and a pyrimidine type. As known enzymes of purine type, there can be mentioned, for example, purine nucleoside phosphorylase (EC 2.4.2.1) and guanosine phosphorylase (EC 2.4.2.15); as known enzymes of pyrimidine type, there can be mentioned, for example, pyrimidine nucleoside phosphorylase (EC 2.4.2.2), uridine phosphorylase (EC 2.4.2.3), thymidine phosphorylase (EC 2.4.2.4) and deoxyuridine phosphorylase (EC 2.4.2.23).

In the present invention, there is no particular restriction as to the microorganisms having nucleoside phosphorylase activity, as long as the microorganisms express at least one nucleoside phosphorylase selected from the group consisting of purine nucleoside phosphorylase (EC 2.4.2.1), guanosine phosphorylase (EC 2.4.2.15), pyrimidine nucleoside phosphorylase (EC 2.4.2.2), uridine phosphorylase (EC 2.4.2.3), thymidine phosphorylase (EC 2.4.2.4) and deoxyuridine phosphorylase (EC 2.4.2.23).

As preferred specific examples of such a microorganism, there can be mentioned microorganism strains included in Norcardia genus, Microbacterium genus, Corynebacterium genus, Brevibacterium genus, Cellulomonas genus, Flabobacterium genus, Kluyvere genus, Mycobacterium genus, Haemophilus genus, Mycoplana genus, Protaminobacter genus, Candida genus, Saccharomyces genus, Bacillus genus, thermophilic Bacillus genus, Pseudomonas genus, Micrococcus genus, Hafnia genus, Proteus genus, Vibrio genus, Staphyrococcus genus, Propionibacterium genus, Sarcina genus, Planococcus genus, Escherichia genus, Kurthia genus, Rhodococcus genus, Acinetobacter genus, Xanthobacter genus, Streptomyces genus, Rhizobium genus, Salmonella genus, Klebsiella genus, Enterobacter genus, Erwinia genus, Aeromonas genus, Citrobacter genus, Achromobacter genus, Agrobacterium genus, Arthrobacter genus and Pseudonocardia genus.

By examining the molecular biological properties, amino acid sequences, etc. of the nucleoside phosphorylases of the above-mentioned microorganism strains based on the recent progress of molecular biology and genetic engineering, it has become possible and even relatively easy to take out a gene for the protein from a microorganism strain, construct a recombinant plasmid, into which the gene and a regulatory region necessary for expression have been inserted, introduce the plasmid into a particular host, and produce recombinant cells in which the protein has been expressed. In view of such a technical standard, even such recombinant cells obtained by introducing the gene for the nucleoside phosphorylase into a particular host are included in the microorganism used in the present invention, in which nucleoside phosphorylase is expressed.

The regulatory regions defined here include promoter sequences, which include operator sequences for control of transcription, ribosome binding sequences (SD sequences) and sequences for formation of transcription. Concrete examples of the promoter sequences include trp promoter form tryptophan operon, lac promoter form lactose operon, P_{L} promoter and P_{R} promoter from lambda phage, promoters for gluconic acid synthesizing enzyme (gnt) of B. subtilis, alkaline protease promoters (aprs), neutral protease promoters (nprs) and α-amylase promoters (amys). Promoters such as tac promoter, which are modified or designed individually, may also be used. As the ribosome binding sequences, although the sequences originated from E. coli or B. subtilis can be mentioned, this sequence is not specially limited provided that the sequence can have its function in an E. coli or B. subtilis host. A consensus sequence consisting of 4 or more nucleotides in a sequence complementary to the 3' terminal region of 16S ribosome RNA may be synthesized and used. The transcription terminating sequence is not always necessary, but those independent of ρ-factor, such as lipoprotein terminator, trp operon terminator may be used. Regarding the order of arrangement of these regulatory sequences, a promoter sequence, a ribosome binding sequence, a gene encoding the nucleoside phosphorylase and a transcription termination sequence are preferably arranged in this order.

As concrete plasmid mentioned here, pBR322, pUC18, Bluescript II SK(+), pKK223-3, pSC101, each of which has a region to make its self-replication possible in E. coli, pUB110, pTZ4, pC194, ρ-11, φ-1 and φ-105, each of which has a region to make its self-replication possible in B. subtilis. As examples of plasmids, which can replicate themselves in two different hosts, pHV14, YEp7 and pBS7 may be used as a vector.

As the particular host mentioned here, there can be cited Escherichia coli as a representative example, as used in Examples described later. However, the host is not restricted thereto and includes other microorganism strains such as Bacillus genus cells (e.g. Bacillus subtilis), yeasts and actinomycetes.

In the present invention, as the nucleoside phosphorylase or the microorganism having such an enzyme activity, there can be used a commercial enzyme, microbial cells having the enzyme activity, a product obtained by treatment of cells, and an immobilized product thereof. The product obtained by treatment of cells is, for example, acetone-dried cells or a cell disruption product prepared by mechanical disruption, ultrasonic disruption, freeze-thawing, pressure-reduced pressure treatment, osmosis treatment, autolysis, cell wall decomposition or surfactant treatment. The product may, as necessary, be purified by ammonium sulfate precipitation, acetone precipitation, or column chromatography.

In the present invention, the metal cation capable of forming a sparingly water-soluble salt with phosphoric acid ion is selected from sodium, potassium, calcium, magnesium, aluminum, barium, iron, cobalt, nickel, copper, silver, molybdenum, lead, zinc and lithium. Of these, particularly preferred are metal salts which have wide industrial applicability and high safety and which give no adverse effect on a reaction. As examples thereof, there can be mentioned magnesium, calcium ion, barium ion and aluminum ion. The metal cation may be used in combination of two or more kinds as long as the effect of the present invention is not impaired.

In the present invention, the metal cation capable of forming a sparingly water-soluble salt with phosphoric acid can be added to a reaction mixture in the form of a compound of a metal with at least one anion selected from chlorine ion, nitric acid ion, carbonic acid ion, sulfuric acid ion, acetic acid ion, and hydroxyl ion. As specific examples, there can be mentioned calcium chloride, calcium nitrate, calcium carbonate, calcium sulfate, calcium acetate, barium chloride, barium nitrate, barium carbonate, barium sulfate, barium acetate, aluminum chloride, aluminum nitrate, aluminum carbonate, aluminum sulfate, aluminum acetate, sodium hydrogencarbonate, sodium carbonate, sodium hydroxide, potassium hydrogencarbonate, potassium carbonate, potassium hydroxide, lithium hydrogencarbonate, lithium carbonate, lithium hydroxide and magnesium hydroxide.

In the present invention, the metal cation may also be be allowed to be in a reaction mixture in the form of a salt of pentose-1-phosphoric acid. As examples of such a salt, there can be mentioned calcium salt of ribose-1-phosphoric acid, calcium salt of 2-deoxyribose-1-phosphoric acid, calcium salt of 2,3-dideoxyribose-1-phosphoric acid, calcium salt of arabinose-1-phosphoric acid, barium salt of ribose-1-phosphoric acid, barium salt of 2-dideoxyribose-1-phosphoric acid, barium salt of 2,3-dideoxyribose-1-phosphoric acid, barium salt of arabinose-1-phosphoric acid, aluminum salt of ribose-1-phosphoric acid, aluminum salt of 2-deoxyribose-1-phosphoric acid, aluminum salt of 2,3-dideoxyribose-1-phosphoric acid and aluminum salt of arabinose-1-phosphoric acid.

When the salt of a metal cation capable of forming a sparingly water-soluble salt with phosphoric acid ion is put in an aqueous solution of a nucleic acid base or a nucleic acid base analogue and is added to a reaction medium, the amount of the metal cation added to the aqueous solution is suitably 0.5 to 20 moles, preferably 1 to 4 moles per mole of the nucleic acid base or the nucleic acid base analogue.

When the salt of a metal cation is put in an aqueous solution of a nucleic acid base or a nucleic acid base analogue and is added to a reaction medium, the aqueous solution may be added to the reaction medium in one portion to give rise to a reaction, or may be added successively to the reaction medium to give rise to a reaction. Here, "added successively" indicates that a nucleic acid base or a nucleic acid base analogue is dissolved or suspended in an aqueous solution of a salt of a metal cation and is added to a reaction system in two or more portions or continuously, suitably over a period of 1 minute to 24 hours, preferably 30 minutes to 24 hours.

Incidentally, when the components required for a reaction are mixed at one time, there may arise problems such as solidification of the reaction mixture depending upon the use amounts of the components. In such a case, problems of such solidification, etc. can be prevented by adding, after the start of reaction, at least one component selected from (1) pentose-1-phosphoric acid, (2) nucleic acid base or nucleic acid base analogue and (3) metal cation, to an aqueous reaction medium which is in the middle of reaction. Here, "the start of reaction" indicates a state in which at least part of the addition amounts of pentose-1-phosphoric acid and nucleic acid base or nucleic acid base analogue has been added in the presence of nucleoside phosphorylase activity.

Each component added after the start of reaction can be added at one time as mentioned above, or in two or more portions batchwise, or continuously, or in a combination of batchwise addition and continuous addition. When the component added after the start of reaction is more than one, at least one component selected from the above-mentioned three components can be added in portions. That is, when the component added after the start of reaction is more than one, an appropriate addition method can be selected for each individual component.

When the aqueous solution of a salt of a metal cation is used in an admixture with a nucleic acid base or a nucleic acid base analogue, the use amount of the aqueous solution of a salt of a metal cation is suitably such that the amount of the metal cation becomes 0.5 to 20 moles, preferably 1 to 4 moles per mole of the nucleic acid base or the nucleic acid base analogue.

When the compound of a metal cation capable of forming a sparingly water-soluble salt with phosphoric acid is per se sparingly soluble in water, the total amount added by the completion of reaction can be added to an aqueous reaction medium before the start of reaction, and control of the pH of the reaction mixture may not be necessary; therefore, such a compound is convensent for use in the present invention. As representative examples of such a compound, there can be mentioned hydroxides of various metals, particularly magnesium hydroxide.

The aqueous reaction medium used in the present process can be any reaction medium which is composed mainly of water and with which a reaction using an enzyme activity, which is intended by the present invention, proceeds and an effect of metal cation addition is exhibited. As such an aqueous reaction medium, there can be mentioned water or an aqueous reaction medium obtained by adding a water-soluble organic solvent to water. The organic solvent can be exemplified by lower alcohols (e.g. methanol and ethanol), acetone, dimethyl sulfoxide and mixtures thereof. There is no particular restriction as to the amount of the solvent used, and the amount may be such that a reaction proceeds favorably. The amount of the organic solvent relative to water can be preferably 0.1% to 70% by weight. The reaction mixture using such an aqueous reaction medium may be used as a buffer solution in order to secure the stability of reaction. As such a buffer solution, there can be used a known buffer solution which can be used for the enzyme reaction of the present invention.

In the synthesis reaction of nucleoside compound according to the present invention, the reaction conditions such as appropriate pH, temperature and the like can be selected depending upon the kinds and properties of pentose-1-phosphoric acid and nucleic acid base as substrate, nucleoside phosphorylase or microorganism having the enzyme activity, as reaction catalyst, and metal salt for removing phosphoric acid out of reaction system. Ordinarily, however, the pH can be selected in a range of 5 to 11, preferably 7.5 to 10.0, more preferably 8.0 to 10 and the temperature can be selected in a range of the freezing point to 80°C, preferably 10 to 60°C. When the pH is out of the control range, there may be a reduction of the conversion rate due to stability of intended product or substrate, reduction in enzyme activity, no formation of sparingly water-soluble salt with phosphoric acid, etc. When pH change arises in the middle of reaction, it is possible to add as necessary an acid such as hydrochloric acid or sulfuric acid, or an alkali such as sodium hydroxide or potassium hydroxide. Needless to say, pH control of reaction mixture may be unnecessary when a sparingly water-soluble metal compound, particularly a metal hydroxide, is used.

The concentration of pentose-1-phosphoric acid and the nucleic acid base used in the reaction is appropriately about 0.1 to 1,000 mM. The molar ratio of the two is such that the amount of the nucleic acid base is 0.1 to 10 moles per mole of pentose-1-phosphoric acid or its salt. The amount is preferably 0.95 mole or less in view of the reaction conversion.

The reaction time differs depending upon the raw materials, the kind of aqueous solvent and the temperature of reaction, but is generally 1 minute to 78 hours, preferably 30 minutes to 24 hours.

The metal compound capable of forming a sparingly water-soluble salt with phosphoric acid is generally added in an amount of 0.1 to 10 moles, preferably 0.5 to 5 moles per mole of the pentose-1-phosphoric acid used in the reaction. Although, in a reaction under a high concentration, the nucleic acid base as substrate and the nucleoside compound as product may be present in the reaction mixture without being fully dissolved, the present invention is applicable even in such a case.

In the present invention, in order to add at least one component selected from pentose-1-phosphoric acid or its salt, a metal cation capable of forming a sparingly water-soluble salt with phosphoric acid or its salt, and a nucleic acid base to a reaction medium so that no solidification of reaction mixture occurs, the following methods can be mentioned as representative methods. Incidentally, the present invention is not restricted to the following methods and, as long as no solidification of reaction mixture occurs, there is no particular restriction as to the timing and times of addition of at least one component selected from pentose-1-phosphoric acid or its salt, a metal cation capable of forming a sparingly water-soluble salt with phosphoric acid or its salt, and a nucleic acid base.
1. A method comprising the steps of feeding pentose-1-phosphoric acid or its salt and a nucleic acid base or its analogue in one portion to start a reaction, accumulating a nucleoside compound up to an appropriate concentration, and then adding a metal cation capable of forming a sparingly water-soluble with phosphoric acid or a salt thereof, in one portion. In this case, the appropriate concentration of nucleoside compound accumulated is preferably 10 mM or more, more preferably 30 mM or more.
2. A method comprising the steps of feeding pentose-1-phosphoric acid or its salt and a nucleic acid base or its analogue in one portion to start a reaction, and then adding a metal cation capable of forming a sparingly water-soluble with phosphoric acid or a salt thereof, stepwise or continuously so as to match the concentration of nucleoside compound accumulated. In this case, the addition of the metal cation can be started when the nucleoside compound has accumulated in an amount of preferably 10 mM or more, more preferably 30 mM or more.
3. A method comprising the steps of adding pentose-1-phosphoric acid or its salt and a metal cation capable of forming a sparingly water-soluble salt with phosphoric acid or its salt in one portion, and then adding a nucleic acid base or its analogue stepwise or continuously so that no solidification of reaction mixture occurs. In this case, the amount of the nucleic acid base or its analogue added initially is preferably 30 mM or less, more preferably 10 mM or less.
4. A method comprising the steps of adding a nucleic acid base or its analogue and a metal cation capable of forming a sparingly water-soluble salt with phosphoric acid or its salt in one portion, and then adding pentose-1-phosphoric acid or its salt stepwise or continuously so that no solidification of reaction mixture occur. In this case, the amount of the pentose-1-phosphoric acid or its salt added initially is preferably 30 mM or less, more preferably 10 mM or less.
5. A method comprising the steps of adding pentose-1-phosphoric acid or its salt, a metal cation capable of forming a sparingly water-soluble salt with phosphoric acid or its salt, and a nucleic acid base or its analogue, into an enzyme solution stepwise or continuously. In this case, the amount of the pentose-1-phosphoric acid or its salt, or the nucleic acid base or its analogue added at the start of reaction is preferably 30 mM or less, more preferably 10 mM or less.

An ordinary method such as concentration, crystallization, dissolution, electrodialysis, adsorption and desorption by ion exchange resin or active carbon, and the like can be used to isolate the nucleoside compound thus produced from the reaction mixture.

### [Examples]

The present invention is described below by way of Examples and Comparative Examples. However, the present invention is in no way restricted by these Examples, etc.

### (Analytical method)

All the nucleoside compounds produced were quantitatively determined by high-performance liquid chromatography. The conditions of analysis were as follows.
Column: YMC-Pack ODS-A312 150x6.0 mm (I.D.) (YMC Co., Ltd.)
Column temperature: 40°C
Pump flow rate: 0.75 ml/min
Detection: UV·260 nm
Eluate: 10 mM phosphoric acid/acetonitrile = 95/5 (V/V)

### Example 1

A DNA of Escherichia coli chromosome was produced as follows. An Escherichia coli K-12/XL-10 strain (Stratagene Co.) was inoculated in 50 ml of a LB medium and cultured at 37°C overnight. The resulting cells were collected and subjected to bacteriolysis with a solution for the bacteriolysis containing 1 mg/ml of lysozyme. The resulting solution was subjected to a phenol treatment and then to an ethanol treatment by an ordinary method to precipitate a DNA. The DNA precipitate was recovered by winding it round a glass rod, washed and used for PCR.

As primers for PCR, there were used two oligonucleotides having base sequences shown by the following sequence Nos. 1 and 2, designed based on the base sequences of known deoD gene of Escherichia coli [GenBank accession No. AE000508 (code region: base Nos. 11531-12250)] (the oligonucleotides had been synthesized by Hokkaido System Science K.K. on consignment).

Sequence No. 1: gtgaattcac aaaaaggata aaacaatggc

Sequence No. 2: tcgaagcttg cgaaacacaa ttactcttt

These primers have, at around respective 5' terminal and 3' terminal, EcoRI and Hind III restriction endonuclease recognition sequences.

The above-obtained DNA of Escherichia coli chromosome was fully digested by the restriction endnuclease Hind III. 0.1 ml of a PCR mixture containing 6 ng/µl of the digested DNA and 3 µm each of the above primers was subjected to 30 cycles of a reaction (each cycle consisted of denaturation: 96°C for 1 minute, annealing: 55°C for 1 minute, and extension reaction: 74°C for 1 minute).

The reaction product and Plasmid pUC 18 (Takara Shuzo Co., Ltd.) were digested by EcoRI and Hind III and ligated using Ligation High (TOYOBO CO.., LTD.), to obtain a recombinant plasmid and, using the recombinant plasmid, Escherichia coli DH5α was transformed. The transformed strain was cultured in a LB agar medium containing 50 µg/ml of ampicillin (Am) and X-Gal (5-bromo-4-chloro-3-indolyl-β-D-galactoside), to obtain a Am-resistant transformed strain as a white colony.

A plasmid having an intended DNA segment inserted was extracted from the thus-obtained transformed strain and named as pUC-PNP 73. The thus-obtained transformed strain was named as Escherichia coli MT-10905.

The Escherichia coli MT-10905 strain was subjected to shake culture at 37°C overnight in a LB medium containing 50 µg/ml of Am. The resulting culture mixture was centrifuged at 13,000 rpm for 10 minutes to collect cells. The cells were suspended in 10 ml of a 10 mM Tris-hydrochloric acid buffer solution (pH 8.0) and disrupted by an ultrasonic wave. The resulting product was used as an enzyme source.

A reaction mixture (1.0 ml) consisting of 100 mM of di(monocyclohexylammonium) salt of 2'-deoxyribose 1-phosphoric acid (a product of SIGMA), 10 to 80 mM of adenine (a product of Wako Pure Chemical Industries, Ltd., special grade chemical), a 100 mM Tris-hydrochloric acid buffer solution (pH 8.0) and 20 units/ml of the above-obtained purine nucleoside phosphorylase, was subjected to a reaction at 50°C for 20 hours. Eight hours after the start of the reaction, calcium chloride was added so that the amount become 150 mM. The results are shown in Table 1.

**Table 1 (Example 1)**

| Adenine concentration (mM) | State of reaction mixture | Deoxyadenosine concentration (mM) |
|---|---|---|
| 10 | Liquid | 10 |
| 20 | Liquid | 18 |
| 40 | Liquid | 38 |
| 60 | Liquid | 56 |
| 80 | Liquid | 79 |

### Comparative Example 1

Calcium chloride was added simultaneously with the addition of enzyme source, and a reaction was conducted in the same manner as in Example 1. The results are shown in Table 2.

**Table 2 (Comparative Example 1)**

| Adenine concentration (mM) | State of reaction mixture | Deoxyadenosine concentration (mM) |
|---|---|---|
| 10 | Liquid | 10 |
| 20 | Solidification | 19 |
| 40 | Solidification | 39 |
| 60 | Solidification | 58 |
| 80 | Solidification | 78 |

### Comparative Example 2

Adenine (80 mM) was subjected to the same reaction as in Example 1 except that no buffer solution was added. The result is shown in Table 3.

**Table 3 (Comparative Example 2)**

| Adenine concentration (mM) | State of reaction mixture | Deoxyadenosine concentration (mM) |
|---|---|---|
| 80 | Solidification | 29 |

### Example 2

A reaction mixture (1.0 ml) consisting of 100 mM of di(monocyclohexylammonium) salt of 2'-deoxyribose 1-phosphoric acid (a product of SIGMA), 10 to 80 mM of thymine (a product of Wako Pure Chemical Industries, Ltd.), a 100 mM Tris-hydrochloric acid buffer solution (pH 8.0) and 20 units/ml of thymidine phosphorylase (a product of SIGMA) was subjected to a reaction at 50°C for 20 hours. Eight hours after the start of the reaction, calcium chloride was added so that the amount became 150 mM. The results are shown in Table 4.

**Table 4 (Example 2)**

| Thymine concentration (mM) | State of reaction mixture | Thymidine concentration (mM) |
|---|---|---|
| 10 | Liquid | 9 |
| 20 | Liquid | 19 |
| 40 | Liquid | 38 |
| 60 | Liquid | 59 |
| 80 | Liquid | 78 |

### Comparative Example 3

Calcium chloride was added simultaneously with the addition of the enzyme source, and a reaction was conducted in the same manner as in Example 2. The results are shown in Table 5.

**Table 5 (Comparative Example 3)**

| Thymine concentration (mM) | State of reaction mixture | Thymidine concentration (mM) |
|---|---|---|
| 10 | Liquid | 10 |
| 20 | Solidification | 19 |
| 40 | Solidification | 38 |
| 60 | Solidification | 58 |
| 80 | Solidification | 79 |

### Comparative Example 4

A reaction of 80 mM of thymine was conducted in the same manner as in Example 10 with no addition of the buffer solution. The result is shown in Table 6.

**Table 6 (Comparative Example 4)**

| Thymine concentration (mM) | State of reaction mixture | Thymidine concentration (mM) |
|---|---|---|
| 80 | Solidification | 26 |

### Example 3

A reaction mixture (0.5 ml) which consisted of 200 mM of thymine (a product of Wako Pure Chemical Industries, Ltd.), a 200 mM Tris-hydrochloric acid buffer solution (pH 8.0) and 20 units/ml of thymidine phosphorylase (a product of SIGMA), was kept at 50°C. A solution containing 200 mM of di(monocyclohexylammonium) salt of 2'-deoxyribose 1-phosphoric acid (a product of SIGMA) was added every hour after the addition of the enzyme solution, in 5 portions (0.1 ml per portion, total 0.5 ml). As a result, the reaction mixture remained as a liquid.

### Comparative Example 5

A reaction was conducted in the same manner as in Example 3 except that 0.5 ml of the solution containing 200 mM of di(monocyclohexylammonium) salt of 2'-deoxyribose 1-phosphoric acid (a product of SIGMA) was added simultaneously with the addition of the enzyme source with no presence of the Tris-hydrochloric acid buffer solution. As a result, the reaction mixture solidified.

### Example 4

A reaction mixture (0.5 ml) consisting of 200 mM of di(monocyclohexylammonium) salt of 2'-deoxyribose 1-phosphoric acid (a product of SIGMA) 200 mM of a Tris-hydrochloric acid buffer solution (pH 8.0), 20 units/ml of thymidine phosphorylase (a product of SIGMA) and 150 mM of calcium chloride was kept at 50°C. A solution containing 200 mM of thymine (a product of Wako Pure Chemical Industries, Ltd.) was added every hour in 5 portions (0.1 ml per portion, total 0.5 ml). As a result, the reaction mixture remained as a liquid. Comparative Example 6

A reaction was conducted in the same manner as in Example 4 except that 0.5 ml of the 200 mM thymine solution was added simultaneously with the addition of the enzyme source with no presence of the Tris-hydrochloric acid buffer solution. As a result, the reaction mixture solidified.

All the nucleoside compounds produced in the following Examples, etc. were quantitatively determined by high-performance liquid chromatography under the following analytical conditions.
Column: YMC-Pack ODS-A514 300x6.0 mm (I.D.) (YMC Co., Ltd.)
Column temperature: 40°C
Pump flow rate: 1 ml/min
Detection: UV 254 nm
Eluate: Phosphoric acid was added, for pH adjustment to 3.5, to an aqueous solution (2,700 ml) containing 20 mM of ammonium acetate; methanol (300 ml) was added; and mixing and degassing were conducted.
Internal standard substance: Nicotinic acid

### Reference Example 1

### Synthesis of di(ammonium) salt of 2-deoxyribose 1-phosphoric acid

Di(monocyclohexylammonium) salt of 2-deoxyribose 1-phosphoric acid (1 g, 2.42 mmol, a product of SIGMA) was suspended in methanol (10 g). Ammonia gas (0.62 g, 36.5 mmol) was blown thereinto and stirring was conducted at 50°C for 2 hours. The resulting reaction mass was filtered at 30°C, followed by washing twice with methanol (5 g) and drying, to obtain intended di(ammonium) salt of 2'-deoxyribose 1-phosphoric acid (0.57 g) at a yield of 95%.
¹H-NMR (D₂O, 270 MHz) d 5.56 (s,1H), 4.03 (m,2H), 3.52 (dd, J=3.3, 12.2 Hz, 1H), 3.41 (dd, J=5.3, 12.2Hz, 1H), 2.17 (m, 1H), 1.87 (d, J=13.9 Hz, 1H), MS (APCI) m/z 213 (M-H)

### Example 5

### Synthesis (1) of 2'-deoxyguanosine

Di(monocyclohexylammonium) salt of 2-deoxyribose 1-phosphoric acid (3.32 g, 7.72 mmol, a product of SIGMA) was dissolved in water (47.1 g). Thereto was added the enzyme solution (0.1 ml) obtained in Example 1. To the resulting reaction mixture was dropwise added, at 50°C in 1 hour, a solution obtained by dissolving guanine (1 g, 6.62 mmol) in a 4.02 wt. % aqueous sodium hydroxide solution (16.45 g, 16.5 mmol). The resulting reaction mass was subjected to a reaction at the same temperature for 2 hours. Then, the reaction mass was analyzed by HPLC. As a result, intended 2'-deoxyguanosine was obtained at a reaction yield of 80%.

### Example 6

### Synthesis (2) of 2'-deoxyguanosine

The di(ammonium) salt of 2-deoxyribose 1-phosphoric acid (2.07 g, 7.72 mmol) synthesized in Reference Example 1 was dissolved in water (47.1 g). Thereto was added the enzyme solution (0.1 ml) obtained in Example 9. To the resulting reaction mixture was dropwise added, at 50°C in 1 hour, a solution obtained by dissolving guanine (1 g, 6.62 mmol) in a 4.02 wt. % aqueous sodium hydroxide solution (16.45 g, 16.5 mmol). The resulting reaction mass was subjected to a reaction at the same temperature for 2 hours. Then, the reaction mass was analyzed by HPLC. As a result, intended 2'-deoxyguanosine was obtained at a reaction yield of 81%.

### Example 7

### Synthesis (3) of 2'-deoxyguanosine

The di(ammonium) salt of 2-deoxyribose 1-phosphoric acid (2.07 g, 7.72 mmol) synthesized in Reference Example 1 was dissolved in water (47.1 g). Thereto was added the enzyme solution (0.1 ml) obtained in Example 1. To the resulting reaction mixture was dropwise added, at 50°C in 1 hour, a solution obtained by dissolving guanine (1 g, 6.62 mmol) in a 5.6 wt. % aqueous potassium hydroxide solution (16.5 g, 16.5 mmol). The resulting reaction mass was subjected to a reaction at the same temperature for 2 hours. Then, the reaction mass was analyzed by HPLC. As a result, intended 2'-deoxyguanosine was obtained at a reaction yield of 79%.

### Example 8

### Synthesis (4) of 2'-deoxyguanosine

The di(ammonium) salt of 2-deoxyribose 1-phosphoric acid (2.07 g, 7.72 mmol) synthesized in Reference Example 1 was dissolved in water (47.1 g). Thereto was added the enzyme solution (0.1 ml) obtained in Example 1. To the resulting reaction mixture was dropwise added, at 50°C in 1 hour, a solution obtained by dissolving guanine (1 g, 6.62 mmol) in a 2.4 wt. % aqueous lithium hydroxide solution (16.5 g, 16.5 mmol). The resulting reaction mass was subjected to a reaction at the same temperature for 2 hours. Then, the reaction mass was analyzed by HPLC. As a result, intended 2'-deoxyguanosine was obtained at a reaction yield of 96%.

### Example 9

### Synthesis of 2'-deoxyadenosine

The di(ammonium) salt of 2-deoxyribose 1-phosphoric acid (2.0 g, 7.72 mmol) synthesized in Reference Example 1 was dissolved in water (30 g). Thereto was added the enzyme solution (0.05 ml) obtained in Example 1. To the resulting reaction mixture was dropwise added, at 50°C in 1 hour, a solution obtained by dissolving adenine (0.89 g, 6.62 mmol) in a 2.4 wt. % aqueous lithium hydroxide solution (16.5 g, 16.5 mmol). The resulting reaction mass was subjected to a reaction at the same temperature for 2 hours. Then, the reaction mass was analyzed by HPLC. As a result, intended 2'-deoxyadenosine was obtained at a reaction yield of 98%.

### Industrial Applicability

In the present invention, in producing a nucleoside compound from pentose-1-phosphoric acid and a nucleic acid base using nucleoside phosphorylase or a microorganism having the enzyme activity, a metal compound capable of forming a sparingly water-soluble salt with phosphoric acid is added; thereby, the phosphoric acid generated as a by-product is removed from the reaction system and a high reaction yield unobtainable with the prior art can be achieved. Further, it is possible to produce a nucleoside compound at a high yield efficiently without inviting the high viscosity or solidification of reaction mixture. With these advantages, the present invention provides a large cost reduction in industrial application and has a big significance.

When the nucleic acid base or a nucleic acid base analogue is used by being dissolved in an aqueous solution of an alkali such as lithium hydroxide, the reaction procedure is simple.

When, as the compound of a metal cation capable of forming a sparingly water-soluble salt with phosphoric acid ion, magnesium hydroxide is used, there are various advantages such as the following, because magnesium hydroxide has a low solubility in water.
(1) There is substantially no pH change of reaction mixture during reaction; therefore, deactivation of enzyme is prevented (use of water-soluble metal compound other than magnesium hydroxide results in a slight pH shift of reaction mixture to alkaline side during reaction).
(2) There is no increase in salt concentration of reaction mixture, associated with reaction; therefore, hindrance of reaction caused by increase in salt concentration is prevented and further a reaction in high concentration is possible.
(3) Metal cation can be added in one portion, before reaction. Further, use of magnesium ion as a metal cation gives a strikingly improved filtration rate in the step of filtration of reaction mixture after the completion of reaction, which is very useful in industrial application.

## Claims

1. A process for producing a nucleoside compound, which comprises reacting (i) pentose-1-phosphoric acid with (ii) a nucleic acid base or a nucleic acid base analogue in an aqueous reaction medium in the presence of nucleoside phosphorylase activity in the presence of (iii) a metal cation capable of forming a sparingly water-soluble salt with phosphoric acid ion, selected from sodium, potassium, calcium, magnesium, aluminum, barium, iron, cobalt, nickel, copper, silver, molybdenum, lead, zinc and lithium cations, which process is **characterized in that**, after the start of the reaction, namely when at least some of the pentose-1-phosphoric acid and at least some of the nucleic acid base or analogue have been added to the reaction medium and nucleoside phosphorglase activity is present, then at least one component selected from the pentose-1-phosphoric acid, the nucleic acid base or the nucleic acid base analogue, and the metal cation is added to the aqueous reaction medium which is in the midst of the reaction.

2. A process for producing a nucleoside compound according to Claim 1, wherein the pentose-1-phosphoric acid is ribose-1-phosphoric acid or 2-deoxyribose-1-phosphoric acid.

3. A process for producing a nucleoside compound according to Claim 1 or 2, wherein the metal cation capable of forming a sparingly water-soluble salt with phosphoric acid is at least one cation selected from calcium ion, barium ion, aluminum ion, lithium ion and magnesium ion.

4. A process for producing a nucleoside compound according to Claim 3, wherein the metal cation capable of forming a sparingly water-soluble salt with phosphoric acid is added to the aqueous reaction medium in the form of as metal compound with at least one anion selected from hydroxy ion, chloride ion, and anions of nitric, carbonic, sulfuric and acetic acids.

5. A process for producing a nucleoside compound according to Claim 3, wherein the metal cation capable of forming a sparingly water-soluble salt with phosphoric acid is added to the aqueous reaction medium in the form of a salt of pentose-1-phosphoric acid.

6. A process for producing a nucleoside compound according to Claim 1, wherein the addition of the at least one component to the aqueous reaction medium from the start of the reaction to the completion of the reaction is made in two or more portions or continuously and gradually.

7. A process for producing a nucleoside compound according to Claim 1 or 6, **characterized in that,** the amounts of the three components, (i) the pentose-1-phosphoric acid, (ii) the nucleic acid base or the nucleic acid base analogue, and (iii) the metal cation result in increase of viscosity or solidification of the reaction mixture when the three components are added in one portion, but the increase of viscosity or the solidification can be prevented by adding a part or the whole of the amount of at least one component in a portion(s) from the start of the reaction to completion of the reaction.

8. A process for producing a nucleoside compound according to any of Claims 1 to 7, **characterized in that** the nucleic acid base or the nucleic acid base analogue is added in the form of an aqueous alkali solution.

9. A process for producing a nucleoside compound according to Claim 8, **characterized in that** the aqueous alkali solution in which the nucleic acid base or the nucleic acid base analogue is dissolved, is a solution containing at least one of lithium hydroxide, lithium carbonate and lithium hydrogencarbonate.

## Patentansprüche

1. Verfahren zur Herstellung einer Nukleosidverbindung, das umfaßt (i) Umsetzen von Pentose-1-phosphorsäure mit (ii) einer Nukleinsäurebase oder einem Analogen der Nukleinsäurebase in einem wässerigen Reaktionsmedium in Gegenwart von Nukleosidphosphorylaseaktivität in Gegenwart (iii) eines Metallketion, das in der Lage ist, ein schwer wasserlösliches Salz ausgewählt aus Netrium-, Kalium-. Katzium-, Megnesium-. Aluminium-, Berium-, Eison-, Kobalt-, Nickel-, Kupfer-, Silber-. Molybdän-, Blei-, Zink- und Lithiumkationen mit einem Phosphorsäureion zu bilden, das Verfahren ist **dadurch gekennzeichnet, daß**
nach dem Start der Reaktion, wenn nämlich mindestans ein Teil der Penlose-1-phosphorsäure und mindestens ein Teile der Nukleinsäurebase oder eines Analogen der Nukleinsäurobase zu dem Reaktionsmedium gegeben wurde und Nukleosidphosphorylaseaktivität vorhanden ist, dann wenigstens eine Komponente ausgewählt aus Pentose-1-phosphorsäure, Nuklefnsäurebase oder der Analogen der Nukleinsäurebase und das Metallkation zu dem wässerigen Reaktionsmedium, das sich in der Mitte der Reaktion befindet, hinzugegeben wurde.

2. Verfahren zur Herstellung einer Nukleosidverbindung nach Anspruch 1, bei dem die Pentose-1-phosporsäure eine Ribose-1-Phosphorsäure oder 2-Desoxyribose-1-phosphorsäure ist.

3. Verfahren zur Herstellung einer Nukleosidverbindung nach Anspruch 1 oder 2, bei dem das Metallkation, das in der Lage ist, ein schwer wasserlösliches Salz mit der Phosphorsäure zu bilden, mindestens ein Kation ausgewähit aus Kalziumion, Bariumion, Aluminiumion, Lithiumion oder Magnesiumion ist.

4. Verfahren zur Herstellung einer Nukleosidverbindung nach Anspruch 3, bei dem das Metallkation, das in der Laga ist, ein schwer wasserlösliches Salz mit der Phosphorsäure zu bilden, dem wässerigen Reaktionsmedium in der Form einer Metallverbindung zugegeben wird, mit mindestens einem Anion ausgewählt aus Hydroxyion, Chloridion und Anionen von Salpeter-, Kohlen-, Schwefel- und Essigsäure.

5. Verfahren zur Herstellung einer Nukleosidverbindung nach Anspruch 3, bei dem das Metallkation, das in der Lage ist, ein schwer wasserlösliches Salz mit der Phosphorsäure zu bilden, dem wässerigen Reaktionsmedium in der Form eines Salzes von Pentose-1-phosphorsäure zugegeben wird.

6. Verfahren zur Herstellung einer Nukleosidverbindung nach Anspruch 1, bei dem die Zugabe der mindestens einen Komponente zu dem wässerigen Reaktionsmedium vom Beginn der Reaktion bis zur Beendigung der Reaktion in zwei oder mehreren Portionen oder kontinuierlich und schrittweise durchgeführt wird.

7. Verfahren zur Herstellung einer Nukleosidverbindung nach einem der Ansprüche 1 bis 6, **dadurch gekennzeichnet, daß**
die Mengen der drei Komponenten (i) der Pentose-1-phosphorsäure, (ii) der Nukleinsäurebase oder dem Analogen der Nukeinsäurebase und (iii) dem Metallkation zu einem Anstieg der Viskositët oder zum Erstarren der Reaktionsmischung führt, wenn die drei Komponenten in einer Portion zugegeben werden, aber der Anstieg der Viskosität oder das Erstarren kann durch die Zugabe eines Teils oder der Gesamtmenge wenigstens einer Komponente in einer Portion vom Beginn der Reaktion bis zur Beendigung der Reaktion verhindert werden.

8. Verfahren zur Herstellung einer Nukleosidverbindung nach einem der Ansprüche 1 bis 7, **dadurch gekennzeichnet, daß**
die Nukleinsäurebase oder das Analoge der Nukleinsäurebase in Form einer wässerigen Alkalilösung zugegeben wird.

9. Verfahren zur Herstellung einer Nukleosidverbindung nach Anspruch 8, **dadurch gekennzeichnet, daß**
die wässerige Alkalilösung, in der die Nukleinsäurebase oder das Analoge der Nukleinsäunebase gelöst sind, eine Lösung ist, die mindestens ein Lithiumhydroxid, Lithiumcarbonat oder Lithiumhydrogencarbonst enthält.

## Revendications

1. Procédé de production d'un composé nucléoside, qui comprend la réaction (i) de l'acide pentose-1-phosphorique avec (ii) une base d'acide nucléique ou un analogue de base d'acide nucléique dans un milieu réactionnel aqueux en présence d'une activité de nucléoside phosphorylase en présence de (iii) un cation métallique capable de former un sel peu soluble dans l'eau avec un ion d'acide phosphorique, choisi parmi les cations de sodium, de potassium, de calcium, do magnésium, d'aluminium, de baryum, de fer, de cobalt, de nickel, de cuivre, d'argent, de molybdène, do plomb, de zinc et de lithium, procédé qui est **caractérisé en ce que,** après le début de la réaction, à savoir lorsqu'au moins une partie de l'acide pentose-1-phosphorique et au moins une partie de la base d'acide nucléique ou de l'analogue ont été ajoutées au milieu réactionnel et lorsque l'activité de nucléoside phosphorylase est présente, alors au moins un composant choisi parmi l'acide pentose-1-phosphorique, la base d'acide nucléique ou l'analogue de base d'acide nucléique, et le cation métallique est ajouté au milieu réactionnel aqueux qui est au milieu de la réaction.

2. Procédé de production d'un composé nucléoside selon la revendication 1, dans lequel l'acide pentose-1-phosphorique est l'acide ribose-1-phosphorique ou l'acide 2-désoxyribose-1-phosphorique.

3. Procédé de production d'un composé nucléoside selon la revendication 1 ou 2, dans lequel le cation métallique capable de former un sel peu soluble dans l'eau avec l'acide phosphorique est au moins un cation choisi parmi l'ion calcium, l'ion baryum, l'ion aluminium, l'ion lithium et l'ion magnésium.

4. Procédé de production d'un composé nucléoside selon la revendication 3, dans lequel le cation métallique capable de former un sel peu soluble dans l'eau avec l'acide phosphorique est ajouté au milieu réactionnel aqueux sous la forme d'un composé métallique avec au moins un anion choisi parmi un ion hydroxy, un ion chlorure et les anions des acides nitrique, carbonique, sulfurique et acétique.

5. Procédé de production d'un composé nucléoside selon la revendication 3, dans lequel le cation métallique capable de former un sel peu soluble dans l'eau avec l'acide phosphorique est ajouté au milieu réactionnel aqueux sous la forme d'un sel de l'acide pentose-1-phosphorique.

6. Procédé de production d'un composé nucléoside selon la revendication 1, dans lequel l'addition dudit au moins un composant au milieu réactionnel aqueux depuis le début de la réaction jusqu'à la fin de la réaction est réalisée en deux parties ou plus ou en continu ou progressivement.

7. Procédé de production d'un composé nucléoside selon la revendication 1 ou 6, **caractérisé en ce que** les quantités des trois composants, (i) l'acide pentose-1-phosphorique, (ii) la base d'acide nucléique ou l'analogue de base d'acide nucléique et (iii) le cation métallique, conduisent à une augmentation de la viscosité ou à la solidification du mélange réactionnel lorsque les trois composants sont ajoutés en une fois, mais l'augmentation de la viscosité ou la solidification peut être empêchée par addition d'une partie ou de la totalité de la quantité d'au moins un composant en une ou plusieurs parties depuis le début de la réaction jusqu'à la fin de la réaction.

8. Procédé de production d'un composé nucléoside selon l'une quelconque des revendications 1 à 7, **caractérisé en ce que** la base d'acide nucléique ou l'analogue de base d'acide nucléique est ajouté sous forme d'une solution aqueuse alcaline.

9. Procédé de production d'un composé nucléoside selon la revendication 8, **caractérisé en ce que** la solution aqueuse alcaline dans laquelle est dissout la base d'acide nucléique ou l'analogue de base d'acide nucléique est une solution contenant au moins de l'hydroxyde de lithium, du carbonate de lithium et de l'hydrogénocarbonate de lithium.
